# EUROPEAN PATENT SPECIFICATION

(11) **EP 4 037 573 B1**
(45) Date of publication and mention of the grant of the patent: **19.07.2023**
(21) Application number: 20793802.8
(22) Date of filing: 28.09.2020
(51) Int. Cl.: A61B 10/04

(54) **MEDICAL INSTRUMENT FOR TAKING TISSUE SAMPLES IN THE BODY**
MEDIZINISCHES INSTRUMENT ZUR ENTNAHME VON GEWEBEPROBEN IM KÖRPER
INSTRUMENT MÉDICAL POUR PRÉLEVER DES ÉCHANTILLONS TISSULAIRES DANS LE CORPS

(30) Priority: 03.10.2019 BE 201905649
(43) Date of publication of application: 10.08.2022
(73) Proprietor: Janssens, Jacques Philibert, 3500 Hasselt (BE)
(72) Inventor: Janssens, Jacques Philibert, 3500 Hasselt (BE)
(74) Representative: Jacobs, Tinneke Ivonne C
(86) International application number: PCT/IB2020/059034
(87) International publication number: WO 2021/064541

(56) References cited:
- WO-A1-2004/006789
- US-A1- 2011 224 577
- US-A1- 2014 171 826
- US-A1- 2014 276 051

## Description

The present invention relates to a medical instrument for taking a tissue sample in the body.

In particular, the invention is intended for a medical instrument which is composed of at least two tubes which fit into each other, respectively an inner tube and an outer tube which is mounted axially slideable over the inner tube, whereby the inner tube possesses a distal end which in its extension is provided with a cutting spiral and a proximal end to turn the spiral in the tissue to be sampled and the outer tube possesses a distal end with a cutting edge to cut loose the tissue in the spiral by sliding the outer tube over the spiral (5).

When using the instrument the distal end with the tool is inserted via a small incision in the body or via an endoscope to the location needing treatment, while the proximal end sticks out of the body and serves to operate the instrument.

The current instruments are not flexible and the positioning of the tool is via a CT scan or other imaging.

An example of such instrument is described in the EP 2.623.036 of the same inventor for an instrument for taking a tissue sample whereby the instrument is composed of two concentric tubes, respectively an outer tube with a cutting distal end and an inner tube which is rotatably and slideably mounted in the outer tube and which is provided with a spiral-shaped tool which is rotated in the tissue to be sampled. Subsequently, the outer tube is rotated over the spiral-shaped tool to obtain the tissue sample.

Rotating the spiral-shaped tool requires a certain torsional force to penetrate into the tissue.

Another instrument is known from US2016/0331878 with a rigid outer tube and a rigid tube fitting therein, the distal end of which is flexibly executed as a wire-wound spring for aspirating bone marrow via a predrilled hole and for following the form of the bone marrow canal. By rotating the inner tube in a counter direction, the wire windings close to form a canal along which the bone marrow can be aspirated.

The flexible wire-wound end has no penetration capacity to penetrate the tissue such that no high torque is needed at the distal end of the inner tube. Both the outer and the inner tube are rigid with the exception of the wire-wound section which can protrude about five centimetres from the outer tube.

Other applications are also known such as the use of catheters or the like which are flexibly bendable and are inserted by means of an endoscope which is internally provided with a working channel in which the catheter is slid.

The endoscope itself is also variably flexible over the length and at its distally oriented head is provided with an ultrasound probe such as in US2014/276051 or with lighting and with a digital camera with which the surgeon can examine the location to be treated and can visually follow and lead the actions to be carried out by manipulating the endoscope and the catheter.

Said US2014/276051 talks of a flexible needle of limited length provided with slits to inject a substance which can destroy tissues such as those of a tumour.

US2014171826 and WO2004006789 disclose a medical instrument provided with a penetrating tool at the distal end of a an internal tube which is flexible and is slidable arranged within an outer tube. The tool is designed to puncture the tissue to be sampled.

It is a current requirement that, if one wants to use an instrument such as that of the EP 2.623.036 via the internal working channel of a flexible endoscope, said instrument must also have a certain flexibility in terms of bending, preferably must have a greater flexibility than that of the endoscope to not counteract the maximum bend of the endoscope. Moreover, the flexibility must be variable over the length and paired, i.e. in both tubes simultaneously and to the same extent.

The higher the requirements of flexibility are however, the lower the torsional rigidity and in other words the smaller the torsional torque becomes that can be transmitted to the tool and this even more so the smaller the diameter of the instrument that must fit in the working channel is and the longer the length of the working channel.

The internal diameter of the internal channel of the current endoscopes is typically restricted to a few millimetres.

To date, it has not been possible to develop an instrument such as that of EP 2.623.036 which fits in a working channel of said small diameter and which unites a sufficient flexibility with a sufficient torsional rigidity to allow taking a tissue sample. The tissue sample is preferably taken intact (biopsy) unlike aspirate (cytology).

The purpose of the present invention is therefore to develop a new medical instrument that does offer this possibility.

To this end, the invention relates to a medical instrument for taking a tissue sample in the body, whereby the instrument is composed of at least two tubes which fit into each other, respectively an inner tube and an outer tube, the inner tube being slideably and rotatably mounted in the outer tube, whereby the inner tube possesses a distal end which in its extension is provided with a cutting and rotationally penetrating spiral and a proximal end to turn the spiral in the tissue to be sampled and the outer tube possesses a distal end with a cutting edge to cut loose the tissue in the spiral by sliding the outer tube over the spiral, characterised in that both tubes over at least an overlapping axial length of 30 cm are executed flexibly bendable because over said length the tubes are provided with one or more grooves which extend through the wall of the tubes and which open upon torsion in one direction and thus increase the bendability relative to the resting position without torsion and close upon torsion in the other direction, such that the torsional rigidity is maximal or increases to a maximum with an increasing torsional force for drawing the cutting spiral like a cork screw into the tissue to be sampled upon rotation of the spiral.

Thus the bendability and the torsional rigidity of the tubes can be paired and variably adjusted over the length by a simple rotation in the one or in the other direction, whereby in one direction a greater torsional force can be transmitted to the tool, for example to turn the cutting spiral-shaped biopsy tool of EP 2.623.036 in the tissue, after which the outer tube pushes with its cutting end over the spiral to cut loose the tissue in the spiral and subsequently, by a rotation of the tubes in the other direction, the flexibility can be increased for withdrawing the instrument with the tissue sample from the body.

The use of a cutting spiral for taking a tissue sample is more advantageous than the use of a needle; because on rotation the spiral draws itself into the tissue like a cork screw, less force is needed compared to a needle. Because the spiral draws itself into the tissue the unwanted "push-back" phenomenon of a needle is also avoided whereby the tissue to be sampled is pushed away and the needle is pushed back as a reaction.

Moreover, this technique allows a complete and intact tissue sample to be taken.

The intention is that the instrument must be able to be relatively long with a length of for example one metre or longer and thin with a diameter of, for example 3 mm or less, and despite this must be able to transmit a torque over said length to the spiral-shaped tool to take a sample and still also offer sufficient pressure and tensile resistance.

The flexibility in the overlapping zones of both tubes must be aligned to each other to allow the tubes to be axially slideable and rotatable around their longitudinal axis relative to each other, also when the instrument is bent in curves to guide the cutting spiral to the location of the tissue where the sample is taken, for example via the airways.

Preferably, the groove patterns in the overlapping flexible lengths of the tubes are aligned to each other such that in said zones the flexibility of both tubes is equal or practically the same and with a simultaneous rotation also change together.

In this way the flexibility of one tube is not counteracted by the lesser flexibility of the other tube.

Preferably, the groove pattern in the overlapping flexible lengths in both tubes (2, 3) is similar or distributed in the same way or even, but not necessarily, practically identical apart from the slight difference in outer diameter.

Consequently, a same flexibility can be obtained as with a spiral spring or with woven wires, but with a greater torque transmission.

Preferably, there are two or more parallel primary grooves in every tube which are uniformly distributed over the contour of the tubes and which are essentially axially oriented, which means that in the axial direction they enclose an acute angle which is less than 45°, preferably less than 30° and even more preferably less than 15°

Such paired and predominantly axial grooves in the tubes are relatively easy to realise, for example by means of laser cutting, with which very narrow grooves can also be realised.

The thinner the grooves, the faster they are closed when rotating in one direction and therefore the faster the torsion is built up.

Preferably, the width of the grooves is less than a few hundreds of a millimetre, preferably of the order of magnitude of one hundredth of a millimetre.

According to a practical embodiment the concerning tubes are preferably manufactured from non-magnetic elastic medical stainless steel. This allows very fine grooves to be realised, for example by means of laser cutting.

Different forms of paired grooves are possible, each with a specific effect on the bend and torsional rigidity. In this way a distinction can be made between primary grooves which run across the flexible section over longer lengths and can circumferentially go around the tube, the axial vector of which is greater than the circumferential one and secondary grooves which give bulges on the primary grooves. In this way tertiary and subsidiary grooves can also be identified.

A primary groove with a spiral-shaped or sinus-shaped cut with predominantly axial vector in both tubes, will for example allow a flexible bend in both directions with a gradual build up or reduction of the torsional rigidity of the rotation of the tubes in the one or the other direction. Consequently the pairing of the groove patterns is less critical such that the linear shifting of the tubes relative to each other still allows rotation.

A block-shaped or tooth-shaped secondary cut on the other hand has more of an incremental or discontinuous effect as will appear below.

Preferably, the diameter of the outer part of the instrument is less than 2.5 mm, more preferably 2 mm at most, such that it can be used in an endoscope with a working channel of said small diameters. The length of both tubes is more than 50 cm.

With the intention of better showing the characteristics of the invention, a few preferred embodiments of a medical instrument according to the invention for taking tissue samples in the body are described hereinafter by way of an example, without any limiting nature, with reference to the accompanying figures, wherein:
figure 1 schematically shows a view of a medical instrument according to the invention;
figure 2 shows the section of the instrument indicated in figure 1 with F2 and this in a resting position;
figure 3 shows a cross-section according to line III-III in figure 2;
figure 4 shows an alternative embodiment according to the cross-section of figure 3;
figure 5 on a larger scale shows the section indicated in figure 2 with the frame F5 but for an alternative embodiment of the invention;
figure 6 shows the section of figure 5 but in another condition under torsional load, whereby the effect of the torsional load can be observed in the primary and subsidiary grooves;
figure 7 shows a variant of figure 5;
   the figures 8 to 12 show other variants of figure 5, respectively in resting condition for the figures 8, 10 and 11 and under torsional load for the figures 9 and 12.

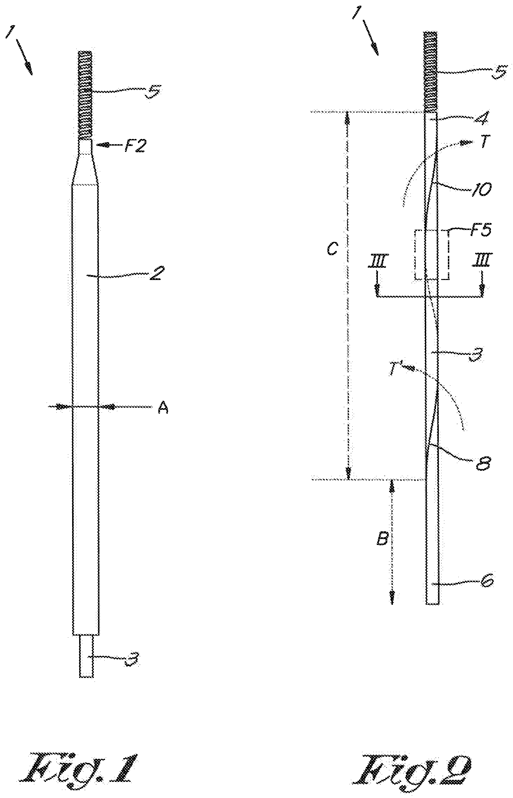

The instrument 1 of figure 1 shown by way of example is an instrument for taking a tissue sample in a patient's body.

The figures are not to scale and are shown out of proportion to better express the dimensional and formal design characteristics.

The instrument 1 is flexibly executed over at least a part C of its length and possesses an outer diameter A which is sufficiently small to be able to fit in a working channel of, for example, less than 2 mm of an endoscope or the like and which must be bendable in curves to, for example, penetrate via the airways into the lungs to locally take a tissue sample there.

The instrument 1 is composed of an outer tube 2 and an inner tube 3 which is slideably and rotatably mounted in the outer tube 2, whereby the outer diameter D of the inner part 3 is slightly smaller than the inner diameter of the outer tube 2 which serves as a kind of sheath for the guidance of the inner tube 3.

Preferably, the tubes 2 and 3 are made of a medical stainless steel or another suitable metal.

At the distal end 4 and in the extension thereof, the inner tube 3 is provided with a cutting and rotationally penetrating spiral 5 for taking a tissue sample.

At its proximal end 6 over a certain length B, the inner tube 3 is rigidly executed to form a sort of handle with which the inner tube 3 can be rotated in to turn the spiral 5 around its shaft in the tissue to be sampled and with which the spiral 5 can be held while the outer tube 3 can be axially moved over the spiral to cut loose the tissue that is grabbed by the spiral 5, for which purpose the outer tube 2 is provided with a cutting edge 7 at the distal end. Alternatively the outer tube 2 can be rotated over the tool 5 to cut off the tissue.

Subsequently, the instrument can be withdrawn from the body to recover the tissue sample.

The section 8 with length C between the rigid proximal end 6 of the inner tube 3 and the tool 5 is flexibly executed because in the case of figure 2 said section is provided with a cut straight through the wall 9 of the inner tube 3 to form a spiral-shaped or sinus-shaped primary groove 10 which extends along the contour and over the entire length C of the flexible part 8 or over a section of said length C, in this case with one and a half winding. The direction of rotation of the spiral-shaped groove is the reverse to the direction of rotation of the spiral-shaped tool.

The primary groove 10 mostly extends in axial direction, more than in lateral direction, whereby the primary groove in the axial direction X-X' encloses an acute angle S which is less than 45°, preferably less than 30° and more preferably less than 15°.

Preferably, the cut is a fine cut with a width E of for example one hundredth of a mm which for example is made with a laser.

The groove 10 forms a means that ensures, when exercising a torsional force T on the proximal end 6 in the opposite direction of rotation of the spiral-shaped groove 10, the groove 10 closes, such that the torsional rigidity increases and is sufficient to turn the spiral-shaped tool 5 into the tissue.

When exercising a torsional force T' in the opposite direction of rotation the groove 10 opens, such that the whole assembly becomes less rigid and the bend flexibility increases, to be able to withdraw the inner tube 3 in the outer tube 2 when the instrument 1 is inserted with one or more curves in the patient's body by means of an endoscope or the like.

Analogously, the outer tube 2 in the overlapping flexible section 8' is provided with a primary groove 10.

Preferably, the grooves 10 in the flexible sections 8 and 8' are aligned such that they result in a similar flexibility of both tubes 2 and 3 in said flexible sections.

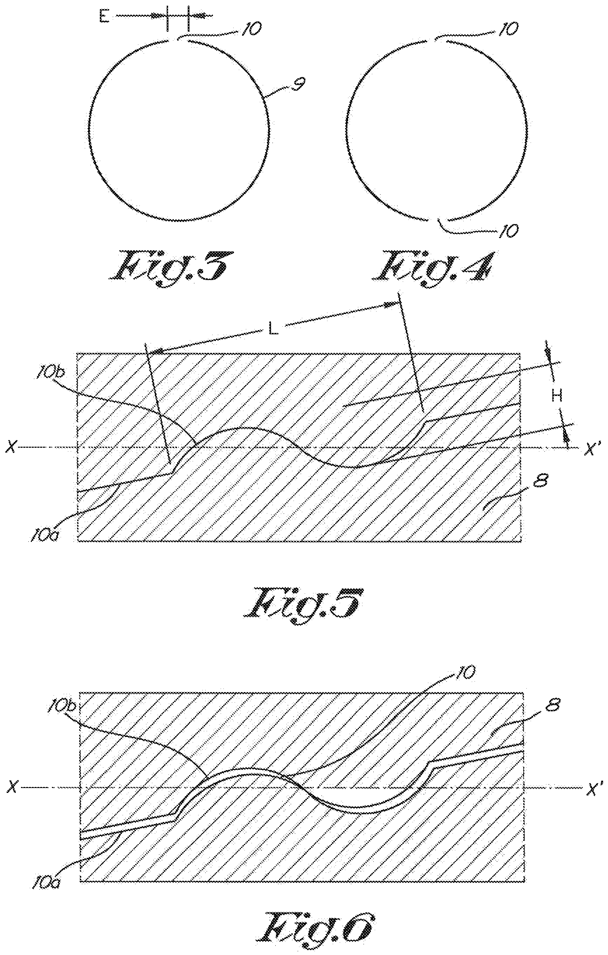

The flexible sections 8, 8' can also be provided with two or more parallel grooves 10 which are uniformly distributed over the contour and thus for example in the case of two grooves 10 are located diametrically opposite each other as shown in figure 4 or in the case four grooves 10 are rotated over an angle of 90°. Consequently the flexibility is increased even more relative to one single groove 10.

Figure 5 shows an example of a groove 10 which is composed of a spiral-shaped primary groove 10a and a sinus-shaped secondary groove 10b grafted thereon formed by a sinus-shaped cut which extends on both sides of the primary cut 10a, said sinus showing an amplitude H and extending over an axial length L of the flexible section 8.

When rotating the rigid proximal end 6 in one direction of rotation the cut closes as shown in figure 5 while the flexible section 8 becomes longer and thinner with a smaller outer diameter D.

In case of a rotation in the opposite direction of rotation the groove 10 opens as shown in figure 6 (schematically strongly exaggerated).

In figure 5, the torsional rigidity and the bend rigidity are maximal, while in figure 6 the flexibility has increased.

The sinus-shaped groove 10b ensures a flexible bend in both directions of the axis X-X'.

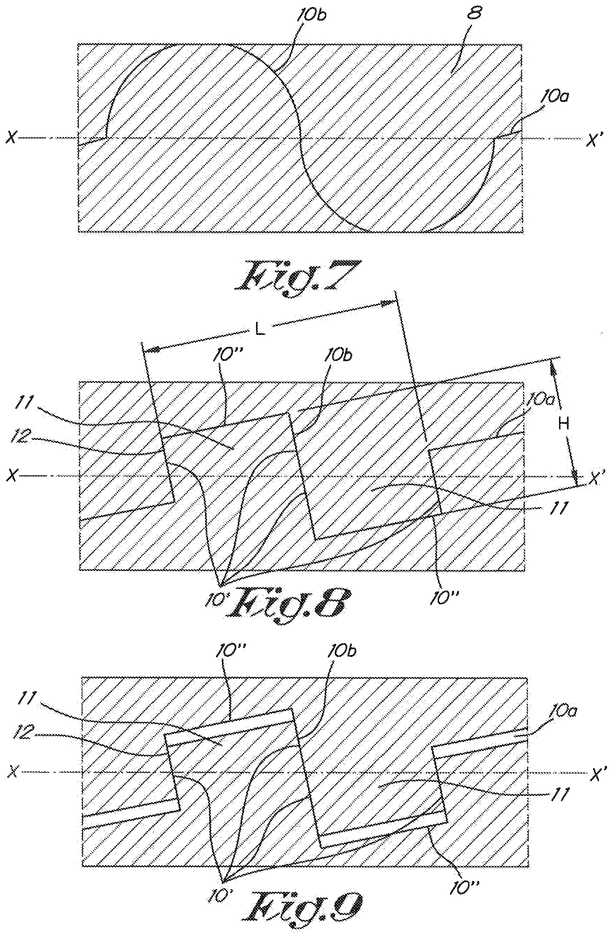

Figure 7 shows a variant of a sinus-shaped groove 10b with a greater amplitude H, whereby the groove 10b extends in circumferential direction in this case over a zone that is greater than half the contour of the inner tube 3.

Figure 8 shows yet another example of a secondary groove 10b which in this case is executed as a block shape and is realised by a cut with U-shaped sections which extend alternatingly on both sides of the primary groove 10a with parallel legs 10' which depart from the primary groove 10a and extend perpendicularly to said groove 10a and which are connected by sections 10" at a distance from the groove 10a.

In this way straight block-shaped teeth 11 are cut out as it were which fit in rectangular recesses 12, obtained by the U-shaped cuts 10'-10" in the wall 9 of the tubular inner tube 2.

In terms of opening and closing upon torsion, the behaviour of the inner tube 3 is analogue as the aforementioned embodiment, whereby figure 8 illustrates the condition upon torsion in one direction with closed groove 10 and figure 9 shows the condition upon torsion in the other direction with an open groove 10.

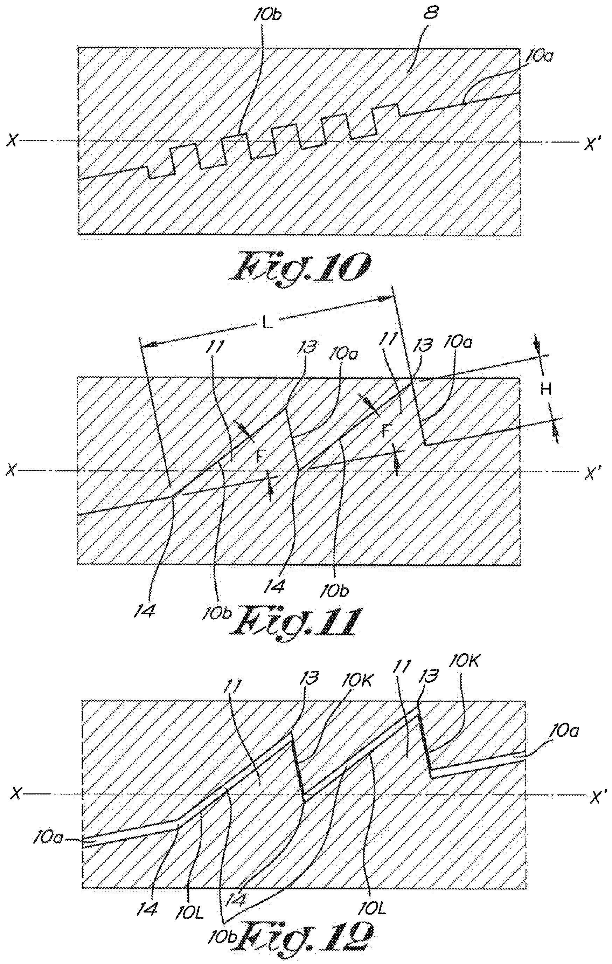

Figure 10 shows an embodiment of a tube with small teeth 11. If the teeth 11 are smaller than the width of the groove 10 when the groove 10 opens a gradual effect can be obtained when the groove 10 opens because the teeth on one side of the line X-X' are than staggered relative to the recesses on the other side of the line X-X'.

In this way the flexibility and the torsional rigidity can also be gradually maintained, also when the torsional force falls away.

Such small toothing 11 can for example also be superimposed on the sinusoidal cut of figure 6.

Yet another type of groove is shown in figure 11, in which a toothed secondary groove 10b is shown which is obtained by a cut with V-shaped sections to form oblique teeth 11 which extend like a sort of sawtooth toothing on one side of a spiral-shaped primary cut 10a and whose tip 13 points away from the primary groove 10a.

In the example shown of figure 11 the V-shaped sections of the groove 10 have a short leg 10K which is perpendicular to the primary groove 10a and a long leg 10L which encloses an acute angle F with the primary groove 10a.

The groove 10 is continuously cut such that the V-shaped sections with said acute angle F point in the same direction of a same end, for example with the tip 14 in the direction of the end 4 with the tool 5.

In this case, when exercising a torsion in one direction, the rotation will occur flexibly thanks to the oblique cut formed by the long leg 10L, but in the event of a torsion in the other direction, the rotation will be abruptly blocked by the straight angle of the teeth 11 formed by the short leg 10K engaging with the recesses 12.

Depending on the direction in which the acute angle F is oriented, in the event of a rotation either the transmitted torsional torque at a maximum value will be maintained at a constant fixed and desired value, or the flexibility will be maintained.

Thus, the tool can, for example, be safeguarded from an overload in case of use that could break or damage the tool 5.

The outer tube 2 can also be made from elastic medical stainless steel and will be executed with one or more primary grooves with which the flexibility and the torsional rigidity can be locally influenced by a torsion in the one or other direction simultaneously and paired with the inner tube.

It is also not excluded that the grooves 10 extend over the entire length B of the flexible part 8, but that in this part 8 shorter grooves are applied which, for example overlap each other lengthways or are positioned in a staggered way such that a variable flexion/torsion is possible.

Different types of grooves 10 can also be combined on the same tube 2 or 3. The sort of grooves is also not restricted to the type as described above.

Preferably, the grooves are such that both tubes 2 and 3 in overlapping flexible zones yield the same flexibility, because the groove pattern is uniformly distributed or similar or even identical, but laterally shifted in the circumferential direction of the tubes 2 and 3.

The present invention is by no means limited to the embodiments described as an example and shown in the drawings, but a medical instrument according to the invention for performing a medical procedure in the body can be realised in all kinds of forms and dimensions, which fall under the scope of the invention which is defined by the appended claims.

## Claims

1. - Medical instrument for taking a tissue sample in the body, whereby the instrument (1) is composed of at least two tubes which fit into each other, respectively an inner tube (3) and an outer tube (2), the inner tube (3) being slideably and rotatably mounted in the outer tube (2), whereby the inner tube (3) possesses a distal end (4) which in its extension is provided with a cutting and rotationally penetrating spiral (5) and a proximal end (6) to turn the spiral in the tissue to be sampled and the outer tube possesses a distal end with a cutting edge to cut loose the tissue in the spiral by sliding the outer tube over the spiral (5), wherein both tubes (2, 3) over at least an overlapping axial length (C) of 30 cm are executed flexibly bendable because over said length the tubes (2, 3) are provided with one or more grooves (10) which extend through the wall (9) of the tubes (2, 3) and which open upon torsion in one direction and thus increase the bendability relative to the resting position without torsion and close upon torsion in the other direction, such that the torsional rigidity is maximal or increases to a maximum with an increasing torsional force for drawing the cutting spiral (5) like a cork screw into the tissue to be sampled upon rotation of the spiral (5).

2. Medical instrument according to claim 1, **characterised in that** in the overlapping flexible lengths the groove pattern in both tubes (2, 3) is such that in said lengths the flexibility of both tubes (2, 3) is equal or practically equal.

3. - Medical instrument according to claim 1 or 2, **characterised in that** in the overlapping flexible lengths the groove pattern in both tubes (2, 3) is similar.

4. - Medical instrument according to any one of the previous claims, **characterised in that** there are two or more parallel primary grooves (10) which are uniformly distributed over the contour of the tubes (2, 3) and which in the axial direction enclose an acute angle which is less than 45°, preferably less than 30° and even more preferably less than 15°.

5. - Medical instrument according to any one of the previous claims, **characterised in that** the concerning tubes (2, 3) show a rigid section without said grooves at the proximal end (6) of the instrument.

6. - Medical instrument according to any one of the previous claims, **characterised in that** both tubes (2, 3) are provided with at least one spiral-shaped primary groove (10) which extends in one or more windings or a section of a winding along the contour of the tubes (2, 3).

7. - Medical instrument according to any one of the previous claims, **characterised in that** both tubes (2, 3) are provided with at least one groove (10) which is composed of a primary longitudinal groove (10a) and secondary grooves (10b) grafted thereon which extend on both sides of the primary groove (10a).

8. - Medical instrument according to claim 6 or 7, **characterised in that** both tubes (2, 3) are provided with at least one primary groove (10a) which is spiral-shaped and runs over equal lengths and with the same pitch in both tubes (2, 3).

9. - Medical instrument according to claim 7 or 8, **characterised in that** both tubes (2, 3) contain one or more sinus-shaped secondary grooves (10b) which extend alternatingly on both sides of a primary groove (10a) in overlapping lengths of both tubes (2, 3).

10. - Medical instrument according to claim 7 or 8, **characterised in that** at least one secondary groove (10b) is a block-shaped groove which extends alternatingly on both sides of a primary groove (10a) with sections departing from the primary groove (10a) perpendicular to said primary groove (10a) and which are connected by sections (10") at a distance from the primary groove (10a) in both tubes simultaneously.

11. - Medical instrument according to any one of the claims 7 or 8, **characterised in that** both tubes (2, 3) contain one or more toothed secondary grooves (10b) in the overlapping lengths which on one side or alternatingly on both sides of the primary groove (10a) can extend with V-shaped sections which with their legs (10L, 10K) extend from the primary groove (10a) and whose tips point away from the primary groove (10a), alternatingly to this and the other side of the primary groove (10a).

12. - Medical instrument according to claim 11, **characterised in that** the V-shaped sections have a long leg (10L) and a short leg (10K), whereby the short leg (10K) is perpendicular to the primary groove (10a) and the long leg (10L) encloses an acute angle (F) with the primary groove (10a).

13. - Medical instrument according to claim 11 or 12, **characterised in that** the V-shaped sections are uniform and with their acute angle (F) are oriented to the same end (4, 6) of the instrument (1).

14. - Medical instrument according to any one of the previous claims, **characterised in that** the width (E) of the grooves (10) is less than a few hundreds of a millimetre, preferably of the order of magnitude of one hundredth of a millimetre.

15. - Medical instrument according to any one of the previous claims, **characterised in that** the outer diameter (A) of the outer tube (2) of the instrument (1) is less than 2.5 mm, is preferably 2.1 mm at most and that the length amounts to more than 50 cm.

16. - Medical instrument according to any one of the previous claims, **characterised in that** the tubes (2, 3) with one or more grooves (10) are made from elastic medical stainless steel.

## Patentansprüche

1. Medizinisches Instrument zur Entnahme einer Gewebeprobe aus dem Körper, wobei das Instrument (1) aus mindestens zwei ineinander passenden Rohren (3), bzw. aus einem inneren Rohr (3) und einem äußeren Rohr (2) besteht, wobei das innere Rohr (3) in dem äußeren Rohr (2) verschiebbar und drehbar gelagert ist, wobei das innere Rohr (3) ein distales Ende (4), das in seiner Erstreckung mit einer schneidenden und drehdurchführenden Spirale (5) versehen ist, und ein proximales Ende (6), das dazu bestimmt ist, die Spirale in dem zu beprobenden Gewebe zu drehen, enthält, und das äußere Rohr ein distales Ende mit einer Schneide enthält, die dazu bestimmt ist, das Gewebe in der Spirale durch Schneiden zu lösen, indem das äußere Rohr über die Spirale (5) geschoben wird, wobei beide Rohre (2, 3) über mindestens eine überlappende axiale Länge (C) von 30 cm flexibel biegbar ausgeführt sind, da die Rohre (2, 3) über die genannte Länge mit einer oder mehreren Rillen (10) versehen sind, die sich durch die Wandung (9) der Rohre (2, 3) erstrecken, und die sich bei der Torsion in eine Richtung öffnen, und somit die Biegsamkeit gegenüber der Ruheposition ohne Torsion erhöhen, und sich bei der Torsion in die andere Richtung schließen, so dass die Torsionssteifigkeit mit zunehmender Torsionskraft maximal ist oder bis einem Maximum zunimmt, um die Schneidspirale (5) wie eine Korkschraube in das zu beprobende Gewebe bei Drehung der Spirale (5) einzuziehen.

2. Medizinisches Instrument nach Anspruch 1, **dadurch gekennzeichnet, dass** das Rillenmuster in beiden Rohren (2, 3) in den sich überlappenden flexiblen Längen derart ist, dass in den genannten Längen die Flexibilität beider Rohre (2, 3) gleich oder praktisch gleich ist.

3. Medizinisches Instrument nach Anspruch 1 oder 2, **dadurch gekennzeichnet, dass** das Rillenmuster in beiden Rohren (2, 3) in den sich überlappenden flexiblen Längen ähnlich ist.

4. Medizinisches Instrument nach einem der vorhergehenden Ansprüche, **dadurch gekennzeichnet, dass** zwei oder mehr parallele primäre Rillen (10) vorhanden sind, die gleichmäßig über die Kontur der Rohre (2, 3) verteilt sind und die in axialer Richtung einen spitzen Winkel von weniger als 45 °, vorzugsweise weniger als 30 ° und noch bevorzugter weniger als 15° umschließen.

5. Medizinisches Instrument nach einem der vorhergehenden Ansprüche, **dadurch gekennzeichnet, dass** die betreffenden Rohre (2, 3) an dem proximalen Ende (6) des Instruments einen starren Abschnitt ohne die genannten Rillen aufweisen.

6. Medizinisches Instrument nach einem der vorhergehenden Ansprüche, **dadurch gekennzeichnet, dass** beide Rohre (2, 3) mit mindestens einer spiralförmigen primären Rille (10) versehen sind, die sich in einer oder mehreren Wicklungen oder in einem Abschnitt einer Wicklung entlang der Kontur der Rohre (2, 3) erstreckt.

7. Medizinisches Instrument nach einem der vorhergehenden Ansprüche, **dadurch gekennzeichnet, dass** beide Rohre (2, 3) mit mindestens einer Rille (10) versehen sind, die aus einer primären länglichen Rille (10a) und sekundären auf diese gepfropften Rillen (10b) besteht, die sich auf beiden Seiten der primären Rille (10a) erstrecken.

8. Medizinisches Instrument nach Anspruch 6 oder 7, **dadurch gekennzeichnet, dass** beide Rohre (2,3) mit mindestens einer primären Rille (10a) versehen sind, die spiralförmig ausgebildet ist und sich in beiden Rohren (2, 3) über gleiche Längen und mit der gleichen Teilung erstreckt.

9. Medizinisches Instrument nach Anspruch 7 oder 8, **dadurch gekennzeichnet, dass** beide Rohre (2, 3) eine oder mehrere sinusförmige sekundäre Rillen (10b) enthalten, die sich abwechselnd auf beiden Seiten einer primären Rille (10a) in sich überlappenden Längen beider Rohre (2, 3) erstrecken.

10. Medizinisches Instrument nach Anspruch 7 oder 8, **dadurch gekennzeichnet, dass** mindestens eine sekundäre Rille (10b) eine blockförmige Rille ist, die sich abwechselnd auf beiden Seiten einer primären Rille (10a) erstreckt, wobei Abschnitte von der primären Rille (10a) senkrecht zu der primären Rille (10a) abweichen und durch Abschnitte (10") im Abstand von der primären Rille (10a) in beiden Rohren gleichzeitig verbunden sind.

11. Medizinisches Instrument nach einem der Ansprüche 7 oder 8, **dadurch gekennzeichnet, dass** beide Rohre (2, 3) eine oder mehrere verzahnte sekundäre Rillen (10b) in den sich überlappenden Längen enthalten, die sich einseitig oder abwechselnd auf beiden Seiten der primären Rille (10a) mit V-förmigen Abschnitten erstrecken können, die sich mit ihren Schenkeln (10L, 10K) von der primären Rille (10a) erstrecken und deren Spitzen von der primären Rille (10a) weg zeigen, abwechselnd zu dieser und der anderen Seite der primären Rille (10a) zeigen.

12. Medizinisches Instrument nach Anspruch 11, **dadurch gekennzeichnet, dass** die V-förmigen Abschnitte einen langen Schenkel (10L) und einen kurzen Schenkel (10K) haben, wobei der kurze Schenkel (10K) senkrecht zu der primären Rille (10a) steht und der lange Schenkel (10L) einen spitzen Winkel (F) mit der primären Rille (10a) umschließt.

13. Medizinisches Instrument nach Anspruch 11 oder 12, **dadurch gekennzeichnet, dass** die V-förmigen Abschnitte einheitlich sind und mit ihrem spitzen Winkel (F) auf dasselbe Ende (4, 6) des Instruments (1) ausgerichtet sind.

14. Medizinisches Instrument nach einem der vorhergehenden Ansprüche, **dadurch gekennzeichnet, dass** die Breite (E) der Rillen (10) weniger als einige hundert Millimeter, vorzugsweise der Größenordnung eines hundertstel Millimeters, beträgt.

15. Medizinisches Instrument nach einem der vorhergehenden Ansprüche, **dadurch gekennzeichnet, dass** der äußere Durchmesser (A) des Außenrohres (2) des Instruments (1) kleiner als 2,5 mm ist, vorzugsweise höchstens 2,1 mm beträgt, und dass die Länge mehr als 50 cm beträgt.

16. Medizinisches Instrument nach einem der vorhergehenden Ansprüche, **dadurch gekennzeichnet, dass** die Rohre (2, 3) eine oder mehrere Rillen (10) umfassen, die aus elastischem medizinischem Edelstahl gefertigt werden.

## Revendications

1. Instrument médical destiné au prélèvement d'un échantillon de tissu dans le corps, dans lequel l'instrument (1) se compose d'au moins deux tubes qui s'insèrent l'un dans l'autre, respectivement un tube interne (3) et un tube externe (2), le tube interne (3) étant monté en coulissement et en rotation dans le tube externe (2), dans lequel le tube interne (3) possède une extrémité distale (4) qui, dans son prolongement, est équipée d'une spirale coupante et pénétrant en rotation (5), et une extrémité proximale (6) destinée à faire tourner la spirale dans le tissu qui doit être échantillonné, et le tube externe possède une extrémité distale qui comprend un bord coupant pour détacher le tissu en le coupant dans la spirale en faisant coulisser le tube externe par-dessus la spirale (5), dans lequel les deux tubes (2, 3), sur au moins une longueur axiale de chevauchement (C) de 30 cm, sont réalisés pour pouvoir fléchir de manière flexible, du fait que, sur ladite longueur, les tubes (2, 3) sont équipés d'une ou de plusieurs rainures (10) qui s'étendent à travers la paroi (9) des tubes (2, 3) et qui s'ouvrent au cours d'une torsion dans une direction en augmentant ainsi la flexibilité par rapport à la position de repos exempte de torsion et se ferment au cours d'une torsion dans l'autre direction, d'une manière telle que la rigidité en torsion est maximale ou augmente jusqu'à un maximum de manière conjointe avec une augmentation de la force de torsion dans le but de tirer la spirale coupante (5) à la manière d'un tire-bouchon dans le tissu qui doit être échantillonné, au cours de la rotation de la spirale (5).

2. Instrument médical selon la revendication 1, **caractérisé en ce que**, dans les longueurs flexibles qui se chevauchent, le modèle de rainure dans les deux tubes (2, 3) est tel que, dans lesdites longueurs, la flexibilité des deux tubes (2, 3) est égale ou pratiquement égale.

3. Instrument médical selon la revendication 1 ou 2, **caractérisé en ce que**, dans les longueurs flexibles qui se chevauchent, le modèle de rainure dans les deux tubes (2, 3) est similaire.

4. Instrument médical selon l'une quelconque des revendications précédentes, **caractérisé en ce que** l'on prévoit deux rainures primaires parallèles ou plus (10) qui sont distribuées d'une manière uniforme sur le contour des tubes (2, 3) et qui, dans la direction axiale, forment un angle aigu qui est inférieur à 45°, de préférence inférieur à 30° et de manière encore plus préférée inférieur à 15°.

5. Instrument médical selon l'une quelconque des revendications précédentes, **caractérisé en ce que** les tubes concernés (2, 3) présentent un tronçon rigide exempt desdites rainures à l'extrémité proximale (6) de l'instrument.

6. Instrument médical selon l'une quelconque des revendications précédentes, **caractérisé en ce que** les deux tubes (2, 3) sont équipés d'au moins une rainure primaire (10) qui présente une configuration en forme de spirale, qui s'étend sous la forme d'un ou de plusieurs enroulements ou un tronçon d'un enroulement sur le contour des tubes (2, 3).

7. Instrument médical selon l'une quelconque des revendications précédentes, **caractérisé en ce que** les deux tubes (2, 3) sont équipés d'au moins une rainure (10) qui se compose d'une rainure longitudinale primaire (10a) et de rainures secondaires (10b) qui viennent s'y greffer, qui s'étendent sur les deux côtés de la rainure primaire (10a).

8. Instrument médical selon la revendication 6 ou 7, **caractérisé en ce que** les deux tubes (2, 3) sont équipés d'au moins une nervure primaire (10a) qui présente une configuration en forme de spirale et qui s'étend sur des longueurs égales et avec le même pas dans les deux tubes (2, 3).

9. Instrument médical selon la revendication 7 ou 8, **caractérisé en ce que** les deux tubes (2, 3) contiennent un ou plusieurs rainures secondaires (10b) qui présentent une configuration de forme sinusoïdale, qui s'étendent en alternance sur les deux côtés d'une rainure primaire (10a) dans des longueurs des deux tubes (2, 3) qui se chevauchent.

10. Instrument médical selon la revendication 7 ou 8, **caractérisé en ce qu'**au moins une rainure secondaire (10b) représente une rainure qui présente une configuration en forme de bloc, qui s'étend en alternance sur les deux côtés d'une rainure primaire (10a), avec des tronçons qui s'écartent de la rainure primaire (10a) perpendiculairement à ladite rainure primaire (10a) et qui sont reliés par des tronçons (10") à une distance de la rainure primaire (10a) dans les deux tubes, de manière simultanée.

11. Instrument médical selon l'une quelconque des revendications 7 ou 8, **caractérisé en ce que** les deux tubes (2, 3) contiennent un ou plusieurs rainures secondaires dentées (10b) dans les longueurs qui se chevauchent, qui, sur un côté ou en alternance sur les deux côtés de la rainure primaire (10a), peuvent s'étendre avec des tronçons qui présentent une configuration en forme de V, avec lesquels leurs branches (10L, 10K) s'étendent à partir de la rainure primaire (10a) et dont les extrémités pointent à l'écart de la rainure primaire (10a), en variante pointent dans la direction de celle-ci et de l'autre côté de la rainure primaire (10a).

12. Instrument médical selon la revendication 11, **caractérisé en ce que** les tronçons qui présentent une configuration en forme de V possèdent une grande branche (10L) et une petite branche (10K), dans lequel la petite branche (10K) est perpendiculaire à la rainure primaire (10a) et la grande branche (10L) forme un angle aigu (F) avec la rainure primaire (10a).

13. Instrument médical selon la revendication 11 ou 12, **caractérisé en ce que** les tronçons qui présentent une configuration en forme de V sont uniformes et sont orientés, avec leur angle aigu (F), dans la direction de la même extrémité (4, 6) de l'instrument (1).

14. Instrument médical selon l'une quelconque des revendications précédentes, **caractérisé en ce que** la largeur (E) des rainures (10) est inférieure à quelques centaines de millimètre, de préférence de l'ordre de grandeur d'une centaine de millimètre.

15. Instrument médical selon l'une quelconque des revendications précédentes, **caractérisé en ce que** le diamètre externe (A) du tube externe (2) de l'instrument (1) est inférieur à 2,5 mm, s'élève de préférence à 2,1 mm au maximum, et **en ce que** la longueur s'élève à plus de 50 cm.

16. Instrument médical selon l'une quelconque des revendications précédentes, **caractérisé en ce que** les tubes (2, 3) qui comprennent une ou plusieurs rainures (10) sont réalisés à partir d'acier inoxydable élastique médical.
